# EUROPEAN PATENT APPLICATION

(11) **EP 0 597 577 A1**
(43) Date of publication of application: **18.05.1994**
(21) Application number: 93306923.9
(22) Date of filing: 01.09.1993
(51) Int. Cl.: C12Q 1/00, G01N 33/49, G01N 33/53, B01D 37/00, G01N 33/86

(54) **Separation of plasma or serum from whole blood using a red blood cell binding component and a polymer containing multiple cationic sites**

(30) Priority: 02.09.1992 US 940266
(71) Applicant: ENZYMATICS, INC., Horsham, Pennsylvania 19044 (US)
(72) Inventor: Daubney, Stephan D., Lansdale, Pennsylvania 19446 (US); Devenny, Sandra G., Wayne, Pennsylvania 19087 (US); Phelps, Donna J., Warminster, Pennsylvania 18974 (US); Johnston, James B., Ambler, Pennsylvania 19002 (US); Palmer, John L., Philadelphia 19118 (US)
(74) Representative: Coxon, Philip

(57) **Abstract**

The present invention relates to a reagent for forming stabilized, clumped red blood cells, comprising a red blood cell binding component and a polycationic polymer component. The invention further relates to a method for separating plasma or serum from whole blood, comprising contacting whole blood with a red blood cell binding component and a polycationic polymer component to form stabilized, clumped red blood cells and then removing the stabilized, clumped red blood cells from the plasma or serum. The invention also relates to a device for separating plasma or serum from whole blood, comprising a clumping layer, which comprises a red blood cell binding component and a polycationic polymer component, a hematocrit reducing layer and a free cell removing layer and a method for using such a device, comprising introducing whole blood into the device and causing the whole blood to pass through the device.

## Description

### FIELD OF THE INVENTION

The present invention relates to a method and device for separating free plasma or serum from whole blood. Red blood cells within a whole blood sample are clumped by a binding component and stabilized by a polymer containing multiple cationic sites. The stabilized clumps are removed from the blood by, for example, a filtration stack containing multiple layers, while the plasma or serum is allowed to pass through the stack. The separated serum or plasma can then be analyzed for determining cholesterol or glucose levels in the blood.

### BACKGROUND OF INVENTION

Many diagnostic devices use serum or plasma as a sample, since the determination of cholesterol or glucose levels in blood is measured in terms of the serum or plasma levels. The presence of red blood cells will interfere in these measurements because red blood cell membranes contain an appreciable concentration of cholesterol, resulting in an inappropriately elevated cholesterol reading.

Capillary measuring devices, which can be used for measuring concentrations of metabolites such as cholesterol, require free liquid plasma or serum. Examples of these devices are described in U.S. Patent 5,032,506 and 5,036,000, and in copending U.S. Patent Application Serial No. 07/500,554, each of which is hereby incorporated by reference. Other devices measuring high density lipoproteins (HDL), triglycerides and other chemical metabolites also rely on free liquid plasma or serum.

In general, most people do not like to give blood. Accordingly, there is an incentive to minimize the amount of blood required for these devices. While it is relatively easy to collect 20, 30 or even 40 microliters of blood from a fingerstick, it becomes increasingly difficult to collect blood as the amount required increases. Collecting blood in excess of approximately 100 microliters is exceedingly difficult. Most diagnostic devices, however, are typically designed for measuring 50% hematocrit blood. Since at this hematocrit level 100 microliters of blood contains only 50 microliters of plasma, a high value is placed on the efficient recovery of plasma from the blood.

A multitude of methods are known for separating red blood cells from whole blood to obtain cell-free serum or plasma. Such methods include centrifugation or allowing the blood to clot and the clot to retract. These methods are either time consuming or require the use of instruments and machinery such as centrifuges. *See,* *e.g*., U.S. Patents 3,882,021 and 3,862,042 to Ayres and U.S. Patent 4,189,382 to Zine.

It is also known to separate red blood cells by filtration or by migration through absorbent pads. Commercial applications that use absorbent pads do not generate free liquid plasma or serum; rather the filtered material is absorbed onto the pad. Osmotic force is exerted on the liquid absorbing onto a hydrophilic pad to facilitate the separation of cellular and liquid components. This force, however, prevents recovery of free liquid plasma from the pad because the absorbent force is not reversible.

Regarding filtration, the patent and nonpatent literature teach the separation of blood using glass fiber filters, silanized glass fiber filters, or filters composed of a combination of cellulose, glass and polyester fibers. *See, e.g.,* U.S. Patents 4,477,575 and 4,816,224 to Vogel et al. Examples of the last combination are commercially distributed by Ahlstrom under the trade name CytoSep®.

While these various filter materials are capable of separating blood to produce free plasma, they do so in very poor yields. For example, a filter stack composed of glass filters can require over 200 microliters of blood before 20 microliters of free plasma could be reliably obtained, giving an efficiency of plasma recovery of only 20%.

This low yield is presumably due to the high concentration of red blood cells in whole blood. The volume fraction of blood occupied by the cellular component (the blood hematocrit value) can range up to 55% by volume, corresponding to about 6 x 10⁹ cells/mL. These high values put an enormous load on such a filter system, leading to rapid clogging of the filter. The usual means to deal with this high load is to increase the area of membrane filters or the volume of depth filters. As filter area or volume increase, however, plasma yield decreases due to the large amount of wettable surfaces that will absorb and retain plasma.

The use of lectin and antibodies to clump red blood cells is also known. *See, e.g.,* Richard D. Leavitt et al., Journal of Biological Chemistry, 252, 2961-2966 (1977), Lawrence W. Allen et al., Medical Sciences, 63, 334-341 (1969), and K. Nassar et al., Experimental Cell Research, 132, 99-104 (1981), each of which is hereby incorporated by reference. The antibodies or lectin clump blood into large multicellular clumps containing greater than 10-20 cells. During filtration, however, these clumps are very sensitive to shear forces, which cause the clumps to break apart and form very small clumps or free red blood cells. As such, the filters are exposed to a load that is only marginally different from that seen in the absence of a hemagglutinating reagent, resulting in low yields similar to those obtained with conventional filtration methods.

### SUMMARY OF THE INVENTION

An object of the present invention is to provide a device and method for producing plasma or serum from whole blood that is free from interference from red blood cells.

Another object of the present invention is to produce efficiently such free plasma or serum in high yield from small quantities of whole blood samples.

In accomplishing these and other objects of the invention, there is provided a reagent for forming stabilized, clumped red blood cells, comprising an amount of a red blood cell binding component effective for clumping the red blood cells and an amount of a polycationic polymer component effective for stabilizing the clumped red blood cells.

There is also provided a method for separating plasma or serum from whole blood, which comprises contacting whole blood with an amount of a red blood cell binding component effective for clumping red blood cells and an amount of a polycationic polymer component effective to form stabilized, clumped red blood cells and then removing the stabilized, clumped red blood cells from the plasma or serum.

There is also provided a device for separating plasma or serum from whole blood, comprising a clumping layer, which comprises a red blood cell binding component and a polycationic polymer component, a hematocrit reducing layer and a free cell removing layer.

### DESCRIPTION OF PREFERRED EMBODIMENTS

In accordance with the present invention, whole blood may be separated by a reagent which forms clumps of red blood cells and stabilizes those clumps for subsequent removal from the blood. The stabilized clumps are resistant to shear and can be removed by filtration without disruption to generate free plasma or serum in excellent yield. Accordingly, the present invention is especially useful in the production of plasma or serum for use in capillary measuring devices.

Clumps of red blood cells are formed by a red blood cell binding component. The red blood cell binding component may be any agent which causes red blood cells to agglutinate. One skilled in the art is aware of the methods which are known for determining the agglutinating ability of an agent. Such methods include, for example, microscopic inspection, alterations in light transmission as a function of cell aggregate size, and titer plate assays.

According to the present invention, preferred red blood cell binding components are lectins. The lectins may be purified before use by any of the known methods for purifying lectins, such as the process disclosed by Rigas and Osgood in *J.* *Biol. Chem.*, **212**, 1955, pp. 607-615. The lectins may also be used without purification, as a crude preparation, in the presence of other proteins.

The lectins may be nonspecific for human blood types, i.e., where there is no specificity for type A, B, AB or O blood or for RH positive or negative cells in the agglutination by the agent. Suitable nonspecific lectins include concanavalin A and lectins obtained from kidney beans *(Phaseolus vulgaris),* jack beans *(Wistaria floribunda),* wheat germ *(Triticum vulgaris),* soybeans *(Glycine max),* or potatoes *(Solanum tuberosum),* and mixtures thereof. Preferred among the nonspecific lectins is kidney bean lectin.

The lectins used as the red blood cell binding component may also be specific for one or more of the human blood types A, B, AB and O, i.e., agglutination will be observed only for those blood groups to which the lectin will bind. Suitable specific lectins for blood type A include lectins obtained from lima beans or the snail *Helix pomatia.* Suitable lectins for blood types A and B include lectin from *Sophora japonica.* Suitable lectins for blood type O include lectins obtained from *Ulex nanus, Ulex europaeus, Lotus* *siliquosus, Lotus tetragonobolus* and the eel *Anguilla* *anguilla.*

Also preferred as the red blood cell binding component of the present invention are antibodies. Preferred antibodies are those which are directed against human red blood cells. The preferred antibodies are also nonspecific and can agglutinate red blood cells without regard to blood type. According to the present invention, the antibodies may be either polyclonal antibodies or monoclonal antibodies or mixtures thereof. Preferred antibodies are the polyclonal antibodies. Any one of the polyclonal antibodies commonly used for blood typing may be used. Particularly preferred among the polyclonal antibodies is rabbit anti-human erythrocyte antibody.

The amount of red blood cell binding component employed according to the present invention can be the minimum concentration needed to show a positive agglutination effect in any of the known methods for determining agglutination or such higher concentrations as may be appropriate. The upper concentration range is limited only by the inhibition of agglutination shown by some binding components at excess concentration. In general, any concentration which elicits substantial agglutination is appropriate. When the red blood cell binding component is a lectin, the concentration employed is preferably within the range of about 0.2 to about 5.0 micrograms per milliliter of whole blood. More preferably, the concentration of lectin is in the range of about 0.4 to 2.0 micrograms per milliliter of whole blood. When the red blood cell binding component is an antibody preparation containing 4.3 milligrams per mL rabbit anti-human erythrocyte IgG, the concentration employed is preferably within the range of about 0.01 to about 1.0 milligrams per mL of whole blood, more preferably within the range of about 0.04 to 0.4 mg/mL.

According to the present invention, the clumps of red blood cells produced using the red blood cell binding component are stabilized with a polycationic polymer. While the present invention in not limited by any particular theory, it is suggested that polycationic polymers stabilize the clumps by forming bridges between the negative charges on different red blood cells.

The polycationic polymer component used for stabilizing the clumped red blood cells may be any polymer having more than one cationic site. One skilled in the art is aware of the methods which may be used to determine the stability of clumped red blood cells. One such method for determining clump stability to shear turbulence is a vortex test. According to this method, whole blood is mixed with reagents and clumped and free red blood cells counted on a hemocytometer. The clumps are then subjected to vortex mixing for about 10 seconds, after which the clumped blood is again examined under a microscope and the clumped and free red blood cells counted. Particularly preferred polycationic polymer components will yield less than 5% free red blood cells after vortexing.

Suitable polycationic polymers are those based on monomers which contain an amine group. Preferred polycationic polymers are those which are cationic in aqueous solution at about neutral pH. These polymers include, for example, poly-L-lysine, poly-D-lysine, poly-D,L-lysine, polyallylamine, polyalanine, poly(N,N-dimethylaminoethylmethacrylate) and its copolymers with methylmethacrylate, polyethyleneimine and mixtures and copolymers thereof. Particularly preferred polycationic polymers are those in which the amine group is derivatized. These polymers include, for example, poly(diallyldimethylammonium chloride) and poly(1,1-dimethyl-3,5-dimethylene-piperidinium chloride). Most preferred among the polycationic polymers is poly(1,1-dimethyl-3,5-dimethylene-piperidinium chloride) (PDDP).

The amount of the polycationic polymer component employed according to the present invention is at least the minimum concentration necessary to stabilize the clumped red blood cells in a vortex test. The amount of polycationic polymer employed is varied based on the total positive charge contributed by the polymer. Preferably, the polycationic polymer component is used in a concentration of 0.2 to 20 mg per milliliter of whole blood. More preferably, the polycationic polymer is present in a concentration of 0.5 to 2 mg/mL.

According to the present invention there is also provided a method of separating plasma or serum from whole blood which comprises contacting whole blood with an amount of a red blood cell binding component effective to clump red blood cells and an amount of a polycationic polymer component effective to stabilize the clumped red blood cells and then removing the stabilized, clumped red blood cells from the plasma or serum.

The determination of the effective amounts of red blood cell binding component and polycationic polymer component is within the skill of the worker in the art. As indicated, the amount of a red blood cell binding component effective to clump red blood cells may be determined by microscopic inspection of the cells after addition of the agent. The amount of a polycationic polymer component effective to stabilize clumped red blood cells may be determined by a vortex test and subsequent microscopic inspection. Other methods for determining the effective amounts are also known to the skilled artisan.

Whole blood may be contacted with a red blood cell binding component and a polycationic polymer component simultaneously or contacted with a red blood cell binding component and subsequently contacted with a polycationic polymer component. In a particularly preferred embodiment, whole blood is first contacted with a red blood cell binding component and allowed to react until about 98% of the red blood cells are clumped and then contacted with a polycationic polymer component and allowed to react until the clumped red blood cells are stabilized. The time for reaction with either component is not critical, provided it is at least sufficient to allow the red blood cells to clump, as determined, for example, by microscopic inspection, and to allow the clumped red blood cells to be stabilized, as determined, for example, by a vortex test.

The stabilized, clumped red blood cells may be removed from the plasma or serum by any of the known methods. Preferably, the stabilized, clumped red blood cells are removed by filtration.

According to the present invention, there is also provided a device for separating plasma or serum from whole blood comprising a clumping layer, a hematocrit reducing layer and a free cell removing layer.

According to the present invention, the clumping layer clumps and stabilizes red blood cells in a whole blood sample and comprises a red blood cell binding component and a polycationic polymer component. Preferably, the red blood cell binding component and polycationic polymer component are contained on or in a substrate layer. The binding component and the polymer component may be used as is or may be lyophilized on the support. In a particularly preferred embodiment, the binding component and polymer component are contained in an open cell polymeric foam. Preferably, the open cell polymeric foams are the polyurethane foams, more preferably, polyester-type polyurethane foams and polyether-type polyurethane foams. A density of from 60 to 80 pores per inch of foam is preferred. A polyester-type polyurethane foam at 60 pores per inch is particularly preferred. This foam is commercially available from Foamex located in Eddyston, PA, and is known as 60 ppi.

In an alternative embodiment, the clumping layer may be two distinct layers, a binding layer comprising the red blood cell binding component and, downstream from the binding layer, a stabilizing layer comprising the polycationic polymer component.

The clumping layer may also contain additives to assist in the clumping and stabilizing of the red blood cells. Such additives include anti-clotting agents, such as heparin and citrate, to prevent the whole blood from clotting, and detergents, to ensure uniform wetting of the foam.

The hematocrit reducing layer removes the stabilized, clumped red blood cells. It is not necessary for the hematocrit reducing layer to be adjacent to the clumping layer, but the hematocrit reducing layer should be downstream of the clumping layer or, in an alternative embodiment, downstream of the stabilizing layer.

The hematocrit reducing layer may be comprised of any material which can retain the stabilized, clumped red blood cells while allowing the plasma or serum to pass through the layer. Preferably, the hematocrit reducing layer is comprised of cellulose acetate fibers. More preferably, the layer is comprised of cross-linked cellulose acetate fibers. Most preferably, the cellulose acetate fibers are crosslinked with triacetin. The cross-linked cellulose acetate fibers preferably contain about 7% to about 12% triacetin, more preferably about 7% to about 8%. Suitable fibers of this type may be obtained commercially from American Filtrona (Virginia). The hematocrit reducing layer may alternatively be comprised of hydrophilic polyester fibers, preferably hydrophilic polyester fibers bound with low molecular weight polyvinyl alcohol.

The free cell removing layer removes the free red blood cells that escaped clumping. It is not necessary for the free cell removing layer to be adjacent to the hematocrit reducing layer, but it should be downstream of the hematocrit reducing layer.

The free cell removing layer, or sub-filter, may be comprised of any material which can retain free red blood cells while allowing plasma or serum to pass through the layer. The free cell removing layer is preferably comprised of glass fibers. Suitable glass fibers are commercially available from Millipore. More preferably, the free cell removing layer is comprised of a combination of cellulose, glass and polyester fibers. Suitable combinations of this type are commercially available from Ahlstrom under the trade name CytoSep®.

In a preferred embodiment of the present invention, the device for separating plasma or serum from whole blood further comprises a clump removing layer. The clump removing layer can be located downstream of the hematocrit reducing layer and upstream of the free cell removing layer, but it is not necessary that the clump removing layer be adjacent to either of these layers. The clump removing layer filters the remaining stabilized, clumped red blood cells that may have passed through the hematocrit reducing layer.

As with the hematocrit reducing layer, the clump removing layer may be comprised of any material which can retain stabilized, clumped red blood cells while allowing the plasma or serum to pass through the layer. Preferably, the clump removing layer is comprised of cellulose acetate fibers, more preferably cross-linked cellulose acetate fibers, and most preferably, cellulose acetate fibers that have been cross-linked with triacetin. The cross-linked cellulose acetate fibers preferably contain about 7% to about 12% triacetin, more preferably about 7% to about 8%. The clump removing layer may alternatively be preferably comprised of hydrophilic polyester fibers, more preferably hydrophilic polyester fibers bound with low molecular weight polyvinyl alcohol.

In a particularly preferred embodiment, the device according to the present invention further comprises a redox active interfering substance removing layer. The redox active interfering substance removing layer removes redox active interfering substances as discussed in U.S. Patent Application Serial No. 07/500,554. The redox active interfering substance removing layer may be comprised of any material which will remove redox active interfering substances from plasma or serum, such as lead (IV) dioxide, cuprous sulfide and silver ferrocyanide. Preferably, the redox active interfering substance removing layer comprises manganese dioxide.

The redox active interfering substance removing layer is preferably located downstream of the clumping layer, or, in an alternative embodiment, of the stabilizing layer, and upstream of the hematocrit reducing layer, but does not have to be adjacent to either layer.

In a more particularly preferred embodiment, the device according to the present invention further comprises both a clump removing layer and a redox active substance removing layer.

In a most particularly preferred embodiment, the device according to the present invention further comprises a capillary measuring device. Examples of suitable capillary measuring devices are described in U.S. Patents 5,032,506 and 5,036,000 and in copending U.S. Patent Application Serial No. 07/500,554. The capillary measuring device is located downstream of the free cell removing layer.

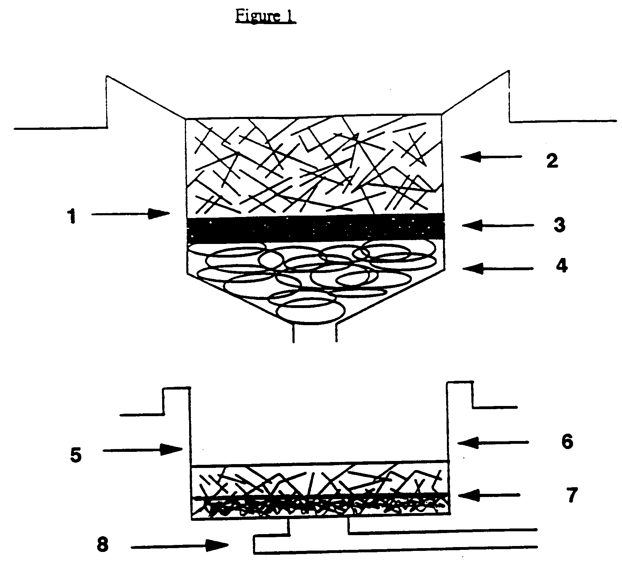

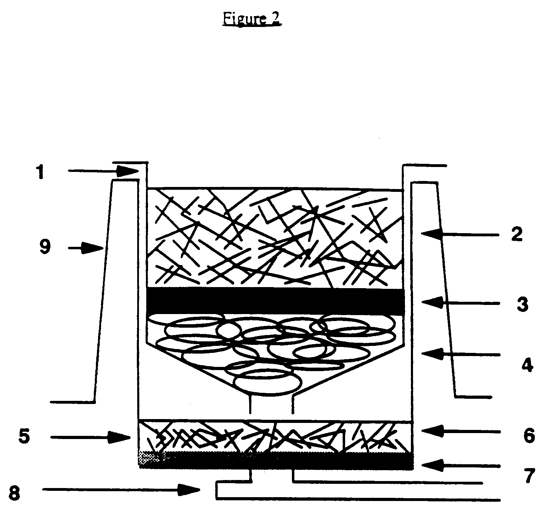

Figures 1 and 2 show two particularly preferred embodiments of the device according to the present invention. A push, pull filter stack is shown in Figure 1, while a push, push stack is shown in Figure 2. Both of these filter stacks are capable of delivering 20µm of free plasma from 60 µm of 50% hematocrit blood. As this blood only contains 30µm total plasma, recovery is at least 66%.

In Figure 1, the upper filter housing, or filter cup, 1 contains three layers. Top layer 2, i.e., the clumping layer, is composed of open cell foam, preferably 0.125 inch high by 0.225 inch diameter polyurethane open cell foam, which contains the red blood cell clumping component, preferably 0.3 to 0.9 mg of crude kidney bean lectin as prepared in Example #1 infra, and the stabilizing polycationic polymer component, preferably 60 to 180 mg of PDDP. Next is the redox active interfering substance removing layer 3, which comprises manganese dioxide powder. This powder is distributed on the hematocrit reducing layer 4, which is preferably a depth filter composed of 7 mg of cross-linked cellulose acetate fibers containing 7% triacetin.

The lower filter housing or filter well 5 contains the clump removing layer 6 and the free cell removing layer 7. The clump removing layer is preferably 2.5 to 4 mg of crosslinked cellulose acetate fibers. The free cell removing layer is preferably a combination of cellulose, glass and polyester fibers from 0.18 to 0.33 millimeters thick.

In operation, whole blood is introduced into the top layer of the upper filter housing and allowed to react with the red blood cell binding component and the polycationic polymer component. A plunger may be used to compress the filter stack causing the stabilized, clumped blood to pass through the stack, and into a capillary measuring device 8.

Figure 2 shows another particularly preferred embodiment of the multilayer device according to the present invention, a push, push filter stack. The filter components are identical to those described in Figure 1, except that the upper filter housing is now seated in a gas-tight sheath 9. Blood is applied to and soaked into the foam, then a plunger is applied to push the clumped blood toward the clump removing filter. The plunger plus upper filter housing now itself becomes a plunger, and after 2-3 seconds this plunger is pushed down to push the plasma into the capillary measuring device.

The following examples are merely illustrative of the invention and should not be construed as limiting. One skilled in the art can make, without undue experimentation, various substitutions and variations and by equivalent means, performing in substantially the same manner, obtain substantially the same results without departing from the teaching and spirit of the invention.

### EXAMPLES

### Example #1 - Preparation of Crude Kidney Bean Lectin

Using the lectin preparation procedure disclosed in D. A. Rigas et al., "Purification and properties of the phytohemagglutinin of *Phaseolus vulgaris" J. Biol. Chem.,* **212**, 1955, p. 607-615, crude PHAP was prepared as follows:
1. 1 kg dried, kidney beans *(Phaseolus vulgaris)* were ground to a fine flour in a mill. This took about 4 passes through the coarse wheels of the electric mill plus about three passes through the fine wheels. There were no large particles left.
2. Using the paddle stirrer at 2-8°C (in the refrigerator), a vortex of pHl H₂SO₄ was generated. Slowly the bean flour was added to the stirring acid and the resulting mixture incubated in a cold room for 16 hours (overnight).
3. The bean sludge was filtered using:
   a. Two layers of nylon bridal veil.
   b. Two layers of gauze (Johnson and Johnson, U.S.P. Type IV, 24 x 20 mesh).
   c. Four layers of gauze.
4. The pH was adjusted to 1.0 with concentrated H₂SO₄.
5. The filtrate was centrifuged at 960 xg (3,000 rpm in GS3 rotor of Sorvall centrifuge) for 60 min. at 2-8°C.
6. The bean material was then filtered through Millipore® Pellicon® cassette system using a 0.45 µm filter cassette. The retentate was recycled into starting material and, after the volume dropped to 200 mL, 500 mL deionized water was added. This process was - repeated three more times.
7. The pH of the filtrate was adjusted to 5.5 with 50% (w/v) NaOH.
8. The filtrate was concentrated using a 30,000 mw filter cassette to approximately 1,000 mL. The concentrated material was then diafiltered by the slow addition of five liters deionized water. Water was added at a rate to keep the retentate concentration at approximately 1,000 mL.
9. A white precipitate formed during the diafiltration step. The mixture was centrifuged at 6,800 xg (8,000 RPM in GS3 rotor) for 60 min. at 2-8°C in GS3.
10. The supernate was carefully decanted, without disturbing the pellet. The pellet was discarded and the activity of the supernate tested by microtitration.
   The Stocks were stored at -20°C, but material in frequent use can be stored at 4-8°C.

### Microtitration Assay of Lectin Agglutinating Activity

### A. Preparation of Formalized Sheep Red Blood Cells

Heparin treated sheep red blood cells were collected by gentle centrifigation and washed twice with a solution containing 75mM NaCl and 75 mM sodium phosphate pH 7.2 (buffer 1). The cells were precipitated by gentle centrifigation between washes at 2,700 x g at 2-8°C. The washed cells were resuspended in buffer 1 to a final concentration of 8% v/v. An equal volume of 3% formalin was added and the cells are fixed by incubation at 37°C overnight with gentle agitation. Following formalin treatment, the cells were again washed four times in buffer 1, as above. The cells were stored at 4°C. The suspension was gently agitated before each use, then diluted four fold to 2% v/v for use in the microtitration assey.

### B. Microtitration Assay

1. The crude lectin stocks were diluted in 0.1 M sodium phosphate pH 7.0 buffer (buffer 2).
2. 40 µL of buffer 2 was added to each well in rows 2-12 of a V-bottom microtiter plate (Dynatech Lab, Inc. Item #001-010-2606). In duplicate, 80 µL of diluted lectin solution was added to row 1 of the plates. This solution was mixed by sucking the mixture up and down in the addition pipette. 80 µL of the diluted lectin was then transferred to row 2. These 2/3 serial dilutions were continued until row 11. The 80 µL removed from row 11 was discarded. Row 12 acted as a negative control.
3. 10 µL of 2% formalized sheep red blood cells were added to each well and each well mixed by gently swirling the plate. The reaction plate was incubated for 2 hours at 25°C. The wells that have complete agglutination were scored (no pellet of RBC's in the bottom of the well visible to the naked eye). The titer of the starting diluted lectin solution is the inverse of the lowest dilution necessary to give complete aggulation.

### Example #2

A preparation of *Phaseolus* *vulgaris* lectin containing 16 micrograms per milliliter of pure lectin was found to be capable of completely agglutinating 2% human O+ erythrocytes in a standard multiwell agglutination assay conducted using 0.01 molar potassium phosphate pH 6.8 +0.9% sodium chloride. This lectin solution retained this activity even when diluted up to 10⁵ fold.

When fresh heparin anticoagulated human blood was diluted 0.9x by addition of the *Phaseolus* lectin to final concentration of 0.5 microgram per milliliter, and incubated 60 seconds, significant visual agglutination occurred. When this agglutinated blood was gently diluted in 10 millimolar phosphate, 0.9% saline pH 6.8, and single erythrocytes were counted under the microscope in a hemocytometer, 3 x 10⁶ single erythrocytes per millimeter of original blood were found. The rest of the erythrocytes, corresponding to approximately 5 x 10⁹ erythrocytes per millimeter original blood, were present as clumps of cells, ranging from two cells per clump up to several hundred cells per clump.

When this same preparation was gently diluted 100-fold into phosphate-saline and then mixed vigorously on a vortex mixer for 10 seconds, the count of single cells rose to 5 x 10⁹ cells per milliliter of blood, i.e., essentially all of the clumps were broken apart by the mechanical agitation. When agitation of lesser severity and duration was employed, a greater portion of cells remained in clumps, demonstrating that the lectin produced a significant agglutinating force, but one that left erythrocytes susceptible to being dislodged from clumps by mechanical forces.

When this experiment was repeated using a crude preparation of *Phaseolus* lectin containing an amount of active lectin equal in agglutinating activity to the preparation above, defined by a well plate agglutination assay, but containing up to 200 times non-lectin protein, essentially the same results were obtained. Likewise, when the amount of active lectin was reduced 4-fold or increased up to 50 fold, essentially the same result was obtained. This showed that increasing lectin could not significantly stabilize clumps against loss of erythrocytes by mechanical dislodgment.

### Example #3

The lectin blood mixture of Example #2 was supplemented with 0.07% PDDP. The count of single cells found in the hemocytometer after 60 seconds incubation corresponded to 4 x 10⁵ single cells per mL of blood. After 10 seconds vortexing of the diluted, agglutinated blood, the single cell count rose to 2 x 10⁸ cells per mL, corresponding respectively to 0.008% non-agglutinated cells before vortexing and 4% after vortexing. Moreover, the same degree of agglutination and mechanical stabilization could be achieved with as little as 10 seconds of incubation of blood with lectin/PDDP reagent at room temperature.

### Example #4

When the experiments of Example #3 were repeated using human blood of blood groups A, B, AB and O, both Rh+ and Rh-, essentially the results were obtained, i.e., there was no blood group specificity to the action of either *Phaseolus* lectin or PDDP.

When rabbit anti-human erythrocyte antibody was substituted for *Phaseolus* lectin, at a concentration of antibody determined from well plate titer assaya to be within the range for effective agglutination of normal human blood, essentially the same results were obtained. Unlike lectin, however, when rabbit anti-human erythrocyte antibody was raised in concentration above the optimum effective concentration, a loss of agglutinating and stabilizing activity was observed. In one case, for instance, an antibody preparation containing 4.3 milligram per milliliter rabbit anti-human erythrocyte IgG effectively agglutinated human erythrocytes at 0.04 to 0.4 milligram per mL, but was ineffective at greater than 1.0 milligram per mL and less than 0.01 milligram per mL. Like the lectin, antibody agglutinated cells did not survive the vortex test in the absence of a polycation. They remained essentially unchanged when 0.05% polyethylene imine, poly-L-lysine, or PDDP was added.

When the stabilizing activities demonstrate in Example #3 were retested substituting the following polymers for PDDP, with the concetration adjusted on the basis of total positive charge contributed, each substance was found to be an effective stabilizer. These substances included polylysine, polyallylamine, and polyethyleneimine.

### Example #5

The lectin/PDDP mixture describe in Example #3 was prepared and then soaked into pieces of polyurethane foam and lyophilized. The dimensions of the foam pieces were 0.193 inches in diameter by 0.170 inches high in order to fit into the barrel of a 1 cc syringe. Each piece of foam contained from 0.3 to 1.2 mg lectin, preferably 0.5 to 0.7 mg, and more preferably 0.6 mg, and from 60 to 180 µg PDDP, preferably 90 to 150 µg, and more preferably 120µg. An amount of fresh, whole blood, from 40 to 80 µL, preferably 50 to 70 µL, and more preferably 60 µL was added to a lectin/PDDP polyurethane foam pellet which had been inserted into the bottom of a 1 cc syringe barrel. The blood was allowed to soak into the foam, then allowed to react to 10 to 60 seconds, preferably 10 to 20 seconds. A plunger was inserted into the barrel to express the agglutinated blood, which was then run through the tests described in Example #3. In the cell-count test, greater than 99% of the red blood cells were clumped. In the vortex test, the single cell count rose to about 3% of the total number of cells, i.e., the clumping and stabilizing agents could be dried onto a support and were effective when blood soaked into the support and the mixture was expressed from it.

In addition, each of the polycations described as substitutes in Example #4 were lyophilized in place of PDDP. The concentrations were adjusted on the basis of total positive charge and then run through the tests described in the current example. For each substituted polycationic polymer, similar agglutination results were obtained.

### Example #6

A 60 ppi polyester-type polyurethane lectin/PDDP containing foam pellet as described in Example #5 was placed in a filter stack, as shown in Figure 1. The foam pellet was placed on top of a cellulose acetate pellet, i.e., the hematocrit reducing layer, comprised of cellulose acetate fibers cross-linked with 8% triacetin. Below this hematocrit reducing layer was an additional cellulose acetate plug, the clump removing layer, which was over a sub-filter of Ahlstrom CytoSep®. The hematocrit reducing layer weighed 2.5 mg. Sixty µL of fresh, whole blood was added to the lectin/PDDP foam, allowed to soak in and react for 10 to 20 seconds. The agglutinated blood was pushed through the foam and hematocrit reducing layer with a plunger and flowed onto the clump removing layer. After 2 to 3 seconds a vacuum was applied which pulled the blood through the clump removing layer and sub-filter. About 15 to 20 µL of plasma was recovered and found to contain no clumps or free cells, and less than 0.2 mg/mL free hemoglobin.

### Example #7

A filter stack was assembled as described in Example #6, but the PDDP was omitted. Sixty µL of fresh, whole blood was added to the lectin foam, allowed to soak in and react for 10 to 20 seconds. The agglutinated blood was pushed through the foam and hematocrit reducing layer with a plunger and flowed onto the clump removing layer. After 2 to 3 seconds a vacuum was applied which pulled the blood through the clump removing layer and sub-filter. Less than 2 µL of clear plasma was recovered, followed by an additional 5 to 8 µL of an extensively hemolyzed solution.

Although preferred embodiments of the invention are described herein in detail, it will be understood by those skilled in the art that variations may be made thereto without departing from the spirit of the invention or the scope of the appended claims.

## Claims

1. A reagent for forming stabilized, clumped red blood cells from a whole blood sample, comprising:
(a) an amount of a red blood cell binding component effective for clumping red blood cells; and
(b) an amount of a polycationic polymer component effective for stabilizing the clumped red blood cells.

2. The reagent of claim 1, wherein said red blood cell binding component is selected from the group consisting of a lectin, an antibody and mixtures thereof.

3. The reagent of claim 2, wherein said lectin is selected from the group consisting of kidney bean lectin, jack bean lectin, wheat germ lectin, soybean lectin, potato lectin, lima bean lectin, *Helix pomatia* lectin, *Sophora* *japonica* lectin, *Ulex nanus* lectin, *Ulex europaeus* lectin, *Lotus siliquosus* lectin, *Lotus tetragonobolus* lectin, *Anguilla anguilla* lectin and mixtures thereof.

4. The reagent of claim 3, wherein said lectin is kidney bean lectin.

5. The reagent of claim 2, wherein said antibody is selected from the group consisting of polyclonal antibodies, monoclonal antibodies and mixtures thereof.

6. The reagent of claim 5, wherein said antibody is rabbit anti-human erythrocyte antibody.

7. The reagent of claim 1, wherein said polycationic polymer component is selected from the group consisting of poly-L-lysine, poly-D-lysine, poly-D,L-lysine, polyallylamine, polyalanine, poly(N,N-dimethylaminoethylmethacrylate), copolymers of N,N-dimethylaminoethylmethacrylate and methylmethacrylate, polyethyleneimine, poly(diallyldimethylammonium chloride), poly(1,1-dimethyl 3,5-dimethylenepiperdinum chloride) and mixtures thereof.

8. The reagent of claim 7, wherein said polycationic polymer component is poly(1,1-dimethyl-3,5-dimethylene-piperdinum chloride).

9. A method for separating plasma or serum from whole blood, comprising:
contacting whole blood with an amount of a red blood cell binding component effective to clump red blood cells and an amount of a polycationic polymer component effective to stabilize the clumped red blood cells; and
removing the stabilized, clumped red blood cells from the plasma or serum.

10. The method of claim 9, wherein said whole blood is first contacted with said red blood cell binding component and then contacted with said polycationic polymer component.

11. The method of claim 9, wherein said removing step is performed by filtration.

12. The method of claim 9, wherein said red blood cell binding component is present in a concentration of from 0.2 to 5 micrograms per mL of whole blood.

13. The method of claim 12, wherein said red blood cell binding component is present in a concentration of from 0.4 to 2 micrograms per mL of whole blood.

14. The method of claim 9, wherein said polycationic polymer component is present in a concentration of from 0.2 to 20 milligrams per mL of whole blood.

15. The method of claim 14, wherein said polycationic polymer component is present in a concentration of from 0.5 to 2 milligrams per mL of whole blood.

16. A device for separating plasma or serum from whole blood, which comprises:
(1) a clumping layer comprising a red blood cell
binding component and a polycationic polymer
component;
(2) a hematocrit reducing layer; and
(3) a free cell removing layer.

17. The device of claim 16, wherein said clumping layer comprises a binding layer comprising a red blood cell binding component and a stabilizing layer comprising a polycationic polymer component.

18. The device of claim 16, wherein said clumping layer further comprises an open cell polyurethane foam selected from the group consisting of polyester-type polyurethane and polyether-type urethane.

19. The device of claim 18, wherein said hematocrit reducing layer comprises cellulose acetate fibers.

20. The device of claim 16, wherein said free cell removing layer comprises cellulose, glass and polyester fibers.

21. The device of claim 16, further comprising a clump removing layer composed of cellulose acetate fibers.

22. The device of claim 16, further containing a redox active interfering substance removing layer of manganese dioxide powder.

23. A method of using the device according to claim 16, comprising:
introducing whole blood into said device; and
causing said whole blood to pass through said device.

24. A device for separating plasma or serum from whole blood, which comprises:
(1) a first filter housing, which contains
(a) a clump removing layer of open cell foam containing a red blood cell binding component and a polycationic polymer component;
(b) a redox active interfering substance removing layer adjacent to said clumping layer containing manganese dioxide powder; and
(c) a hematocrit reducing layer adjacent to said redox active substance removing layer being made of cellulose acetate fibers cross-linked with triacetin; and
(2) a second filter housing, which is or may be brought into fluid communication with said first filter housing, which contains
(d) a clump removing layer being made of cellulose acetate fibers cross-linked with triacetin; and
(e) a free cell removing layer being a combination of cellulose, glass and polyester fibers.

25. The device of claim 24, wherein said first filter housing and said second filter housing are contained in a gas-tight sheath.
